(19) **Europäisches Patentamt**
**European Patent Office**
**Office européen des brevets**

(11) **EP 4 613 176 A1**

(12) # EUROPEAN PATENT APPLICATION

(43) Date of publication:
**10.09.2025 Bulletin 2025/37**

(51) International Patent Classification (IPC):
***A61B 3/11*** *(2006.01)* ***A61B 3/113*** *(2006.01)*

(21) Application number: **24161204.3**

(22) Date of filing: **04.03.2024**

(52) Cooperative Patent Classification (CPC):
**A61B 3/113; A61B 3/112**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Soma Reality GmbH**
**1020 Wien (AT)**

(72) Inventor: **Gollan, Benedikt**
**1020 Wien (AT)**

(74) Representative: **SONN Patentanwälte GmbH & Co KG**
**Riemergasse 14**
**1010 Wien (AT)**

Remarks:
Amended claims in accordance with Rule 137(2) EPC.

(54) **COMPUTER-IMPLEMENTED METHOD FOR MONITORING VISUAL PERCEPTION AND ASSOCIATED COGNITIVE PROCESSING OF A USER**

(57) A computer-implemented method, computer program product and mobile headset for monitoring visual perception and associated cognitive processing of a user, the method comprising: capturing a series of images of an eye; selecting a first subset of the captured images; detecting the position of the pupil in each image in the first subset; selecting a second subset of the captured images; detecting the size of the pupil in each image in the second subset; determining a cognitive load metric based on the detected sizes; determining at least two gaze metrics of an eye gaze behavior based on the detected positions, wherein one of the at least two gaze metrics is a direction of gaze; and determining a measure of targeted attention based on the cognitive load metric and the at least two gaze metrics.

Fig. 1

EP 4 613 176 A1

**Description**

The invention concerns a computer-implemented method for monitoring visual perception and associated cognitive processing of a user, a corresponding computer program product, a mobile headset and one optional use of the disclosed method.

The disclosed method is generally based on obtaining eye tracking data characterizing pupil dilation and point of gaze of the user (an individual). More specifically, the present method comprises: capturing a series of images of an eye; selecting a first subset of the captured images; detecting the position of the pupil in each image in the first subset; selecting a second subset of the captured images; detecting the size of the pupil in each image in the second subset; and determining a cognitive load metric based on the detected sizes.

Methods based on obtaining eye tracking data characterizing pupil dilation and point of gaze of the user are known in the art. For example, US 7438418 B discloses a method for determining one or more of a plurality of predefined mental proficiency levels from the gathered measurement.

Methods monitoring the cognitive load based on pupil dilation are also known in the art. Stolte and Gollan have shown a method for continuous monitoring in their article "Tracking visual search demands and memory load through pupil dilation" (Journal of Vision (2020) 20(6):21, 1-19).

In his doctoral thesis "Sensor-based Online Assessment of Human Attention" Gollan discusses in detail the analysis of different indicators of human attention individually. As a promising approach for future research - without any guidance for an actual technical implementation, even less results - the thesis mentions the combination of analysis of localization of attention via visual attention analysis (eye gaze behavior) and invested attention resources via cognitive load analysis (pupil dilation) and blink rate analysis via a wearable eye tracker as a single sensor.

It is an objective of the present invention to improve on this prior art and provide a technical solution that allows to extend and correlate localized information about user attention with information on cognitive load, thereby providing a localized and qualitative assessment of attention. In other words, the present invention enables a correlation of observed cognitive load to a subset of multiple simultaneous visual signals separated by their location in the view field.

The invention proposes a method as defined in the outset, further comprising: determining at least two gaze metrics of an eye gaze behavior based on the detected positions, wherein one of the at least two gaze metrics is a direction of gaze; and determining a measure of targeted attention based on the cognitive load metric and the at least two gaze metrics.

Capturing images of an eye typically involves controlling a digital camera comprising an image sensor to read out the sensor, often in connection with controlling camera optics in the process. In case images of both eyes of a user shall be captured, separate digital cameras may be foreseen for each eye or one camera may capture an image of both eyes. Optionally, the first subset may include all captured images. The second subset may for example consist of all captured images or the same images present in the first subset, or it may be a different subset.

The measure of targeted attention may be a continuous measure (not limited to discrete levels or categories). The cognitive load metric may be a cognitive load (CL) as defined in previous publications, such as the submission "Demonstrator for Extracting Cognitive Load from Pupil Dilation for Attention Management Services" by Gollan et al (UBICOMP/ISWC '16 ADJUNCT, September 12-16, 2016, Heidelberg, Germany) and the article "Modeling Pupil Dilation as Online Input for Estimation of Cognitive Load in non-laboratory Attention-Aware Systems" by Gollan and Ferscha (COGNITIVE, 2016). Targeted attention can be an indicator of conscious perception. Conscious perception refers to the awareness and subjective experience of sensory stimuli and the processing of information by the conscious mind. It involves the integration of sensory input, interpretation of stimuli, and the formation of a conscious experience. Conscious perception involves being consciously aware of sensory information and experiences. Awareness is fundamental to conscious perception, distinguishing it from unconscious or automatic processing. It implies a subjective and intentional experience of the stimuli. Whereas the reception and neural processing of sensory input generally covers various modalities, including vision, audition, touch, taste, and smell, the present disclosure is focused on the acquisition of visual information, albeit not necessarily limited to conscious perception.

In this method, features like image subset selection may serve to enhance accuracy or efficiency by excluding irrelevant images, while eye tracking data provides insights into the user's visual perception and mental effort allocation. A continuous measure of targeted attention facilitates a more nuanced understanding of cognitive states as compared to discrete levels. The method provides the basis for implementations enabling real-time assessment of user engagement and cognitive workload, contributing to improved human-computer interaction. Another advantage is enhanced comprehension of the user's visual attention distribution, which could inform interface design or content optimization. Finally, the method can enable an objective measurement of cognitive load that can be attributed to a visual context within the entire field of view, enabling more precise evaluation of mental effort required for various tasks. More specifically, the present method can provide a solution to the "Midas Touch Problem", a term coined in the context of HCI, which describes the issue of distinguishing between empty staring (unconscious positioning of the eye during internal thought) and conscious interaction with the environment (voluntary gaze behavior to actively obtain information).

The measure of targeted attention determined according to the disclosed method provides a quantitative and qualitative measure which describes and is at least indicative of the level of awareness and consciousness which has been attributed to a local visual stimulus (e.g. an object) during the course of visual interaction.

For example, the measure of targeted attention may be proportional to the cognitive load metric. This proportionate relationship signifies that an increase or decrease in the cognitive load corresponds to an equivalent change in the measure of targeted attention. Consequently, the user's focus and engagement with specific tasks can be accurately gauged based on their mental effort distribution. This refinement allows for a more precise evaluation of the user's cognitive states and visual attention allocation. In this embodiment, the resulting measure achieves a direct correlation between cognitive load and targeted attention, improving the accuracy in determining user engagement levels and task focus.

In one embodiment, one of the at least two gaze metrics may be a fixation duration, and the measure of targeted attention may be proportional to a step function of the fixation duration with a threshold higher than 50 milliseconds, preferably higher than 200 milliseconds. A fixation is defined as a relatively stable point of gaze on an object or location in the visual scene during which the eye position varies minimally. Fixation duration refers to the length of time that the gaze remains relatively stationary on an object or area, and it may serve as an indicator of attentional processing.

The step function used in this embodiment implies a discontinuous change in the measure of targeted attention at a specific threshold value of the fixation duration. The chosen threshold, which can be above 50 ms, preferably above 200 ms, ensures that only substantial fixations are taken into account, effectively filtering out brief, involuntary eye movements. This approach results in an enhanced signal-to-noise ratio and a more accurate measure of targeted attention. It can allow for a more precise identification of visually attended elements, supported by the consideration of substantial fixations rather than brief, involuntary eye movements. Consequently, this embodiment can exhibit improved sensitivity in assessing user engagement levels with specific tasks or content and enhanced accuracy and reliability in measuring targeted attention, due to the threshold-based step function approach.

Additionally or alternatively, one of the at least two gaze metrics may be a fixation duration, and the measure of targeted attention may be proportional to a sigmoid function of the fixation duration. A sigmoid function can be mathematically represented by an expression such as $(1 - 3^{(-3\,t_i)})$, where $t_i$ denotes the fixation duration. The sigmoid function exhibits a characteristic S-shaped curve, increasing smoothly from zero to one. This smooth increase captures the subtle variations in fixation durations better than a step function, allowing for more nuanced and precise evaluation of targeted attention. The sigmoid function also enables easy thresholding and normalization of attention values by setting upper and lower bounds. This variation of the disclosed method achieves a graceful transition from minimal to maximal attention based on the sigmoid curve, leading to more precise and reproducible evaluation of user engagement levels with specific tasks or content. Also, it can achieve an improved adaptability in handling different ranges of fixation durations and cognitive load levels. Finally, the resulting normalized measure of targeted attention is more easily comparable across various users, tasks, and contexts due to the sigmoid function's upper and lower bounds. It is noted that the step function and the sigmoid function of the fixation duration can both be used at the same time, essentially providing a cut-off below a given threshold and accounting for more subtle variations in a smooth and thus more robust representation above the given threshold.

In another embodiment, one of the at least two gaze metrics may be a count of revisits, wherein a revisit denotes a fixation within a threshold distance of a previous fixation during the complete recorded session. The time window for revisits can span the entire duration of the recorded session, allowing ongoing calculation and continuous update of gaze metrics such as the revisit count as well as saccade efficiency (see below). In this context, a revisit is identified by detecting a fixation that falls within a predefined threshold distance from a previous fixation in the visual scene. This proximity metric helps to quantify user attention allocation and engagement with specific objects or locations. The duration of sessions can have a conditional impact on conscious perception, as it has been observed that extended exposure to stimuli statistically increases awareness. Analyzing revisits in conjunction with other gaze metrics such as fixation duration further improves the representation of cognitive states and provides for enhanced sensitivity in tracking user engagement and attention distribution, facilitated by continuous gaze metric updates during the recorded session.

For example, the measure of targeted attention may be proportional to a sigmoid function of the count of revisits. The sigmoid function may be implemented by a summation formula that may be expressed as sum(1/l), where l ranges from 1 to the detected count of revisits. Optionally, this method may incorporate both, the fixation duration (also as a sigmoid function, as discussed above) and the count of revisits, using a weighted sum. In this instance, a stronger weight may be assigned to the fixation duration term. For instance, the normalized weight of the sigmoid function of the fixation duration may be 60% with the remaining 40% assigned to the term with the sigmoid function of the count of revisits. By combining these gaze metrics with a respective sigmoid function, this variation method enables more accurate assessment of user attention distribution and engagement levels during various tasks or contexts. This approach further improves on the understanding of cognitive load changes by incorporating both fixation duration and revisits measurements. The representation as balanced sigmoid functions enhances adaptability for various tasks or contexts due to the combined impact of these gaze metrics on the targeted attention measure.

In yet another embodiment, one of the at least two gaze metrics may be a measure of clustering of fixations, specifically a nearest neighbor index (NNI) within a time window. Clustering refers to the tendency for fixations to occur near each other, indicating increased attention or focus on specific regions. The NNI is calculated as the average distance from each fixation point to its k-th nearest neighbor fixation within a specified time window (e.g., 30 seconds). Optionally, this method may incorporate additional gaze metrics such as fixation duration and/or count of revisits, allowing for better understanding of user attention distribution and engagement levels during various tasks or contexts.

For example, the measure of targeted attention may be proportional to a sigmoid function of the measure of clustering of fixations (e.g. NNI) within a specified time window. Optionally, this method may incorporate additional gaze metrics such as sigmoid functions of fixation duration and count of revisits. These measures may be combined in a weighted sum, for example, with the strongest weight on the fixation duration term (approximately 50%), secondary weight on the revisit count term (approximately 30%), and lowest weight on the clustering term (approximately 20%).

In another embodiment, one of the at least two gaze metrics may be saccade efficiency. A saccade refers to a rapid eye movement between fixations, typically lasting less than 50 milliseconds. Saccade length can be defined as the distance covered during a saccade, while saccade efficiency can be calculated as the ratio of saccade amplitude (the angular distance between two fixation points) to saccade length. By incorporating saccade efficiency into gaze metrics, this method allows for more comprehensive assessment of user attention distribution and engagement levels. Optionally, the disclosed method includes both fixation-based metrics (such as fixation duration and revisits) and saccade-based metrics (such as saccade efficiency) in a single assessment of targeted attention. This allows for a more comprehensive assessment of user attention distribution and engagement levels during visual tasks or contexts.

For example, the measure of targeted attention may be proportional to saccade efficiency. Additionally, it can be optionally combined with the sigmoid function of fixation duration, the sigmoid function of revisits count, and the sigmoid function of clustering of fixations, forming a weighted sum, for example. The weights for these components may be 40%, 25%, 20%, and 15%, respectively. By incorporating multiple gaze metrics into the targeted attention measure, this method allows for more nuanced measurement of cognitive states by incorporating a multi-faceted targeted attention measure, including saccade efficiency and other gaze metrics.

Optionally, the measure of targeted attention may be aggregated over an integration time window to obtain a field or spatial distribution of targeted attention, for example in the shape of a heatmap. An integration time window is a defined period during which the system collects information about the user's visual perception and cognitive processing. The length of this window can vary depending on the specific task or context and may typically be between 0.5 to 5 seconds. A heatmap represents the intensity or density of a particular variable across a given area, in this case, the targeted attention measure over time. A heatmap can be generated by partitioning the visual scene into a grid or set of regions and calculating the average or accumulated targeted attention value for each region within the integration time window. The resolution of a heatmap is determined by the granularity of the grid used to partition the visual scene. Alternatively, a plurality of individual momentary measurements of targeted attention may be collected and interpolated into a spatial distribution over a scalar field representing the visual scene. These additional method steps provide enhanced representation and potential visualization of user attention distribution and engagement levels over time. They may support the identification of areas that receive more or less focus and be used to identify areas in the visual scene that have captured the user's sustained attention beyond a predefined threshold.

A global maximum of the heatmap or spatial distribution represents the location with the highest targeted attention value during the integration time window. By comparing the global maximum to an attention threshold and associating the current attention target with the location of the global maximum when it is greater than the predefined attention threshold, an embodiment may identify areas in the visual scene where user attention has been significantly focused. For example, a mechanism may be provided for triggering events or alerts based on the duration and intensity of user engagement with specific regions within the visual scene.

By incorporating the methodologies described above into a software application or tool, users can benefit from automated, real-time monitoring and reporting capabilities for better decision-making and optimization. Thereby, the scope of the invention also extends to a computer program product comprising instructions which, when the program is executed by a computer, cause the computer to carry out the method according to any of the embodiments and variations described above.

The computer program product can be delivered in various formats, such as a standalone executable, a library integrated into existing applications, or a cloud-based service accessible via APIs. The invention shall not be limited to any specific hardware configurations or implementations but can instead be adapted to a wide range of use cases and systems. A computer, as the term is used in the present disclosure, refers to a specific, tangible apparatus that is capable of processing data and executing instructions. The computer comprises at least one processor, which is a circuit or chip designed to execute instructions and perform calculations; a memory, such as RAM or ROM, that stores data and instructions for the processor; and input/output devices, such as a keyboard, mouse, display, or other human interface devices that allow the computer to interact with its environment, and specifically one or more users. The computer may also include additional components, such as a storage device (e.g., hard drive, solid-state drive) for long-term data storage, a network interface (e.g., Ethernet port, Wi-Fi adapter) for connecting to a network or the internet, and various other peripherals (e.g., printers, scanners).

The scope of the invention also extends to a mobile headset that incorporates an eye-tracking sensor and a data processing device (generally a computer as defined above) designed to carry out the one or more of the methods described above. The mobile headset may be capable of real-time monitoring, analysis, and feedback of visual perception and cognitive processing, which would make it particularly useful for various applications such as gaming, education, or professional training.

By integrating the eye-tracking sensor and data processing device within a portable and wearable headset, users can benefit from an accessible, easy-to-use platform for monitoring visual perception and cognitive processing, and increased mobility during user engagement with various tasks or contexts, facilitating real-world, ecologically valid assessments. The mobile headset may achieve an enhanced user experience through personalized feedback, alerts, or adaptive content based on the user's visual attention and cognitive states. Moreover, it facilitates improved interaction design for virtual or augmented reality systems by optimizing visual elements and user interfaces to better align with the user's attentional distribution and cognitive processing capabilities.

The mobile headset can be configured as a standalone device or connected to other systems, such as smartphones, tablets, or computers, allowing for flexible integration into existing workflows or applications. The invention shall not be limited to specific hardware configurations or implementations but can instead be adapted to a wide range of use cases and systems.

The preset disclosure also concerns using the methods described above, particularly within the context of a mobile headset, during user interaction with visual content displayed on a computer screen. This specific application provides an efficient and practical solution for monitoring visual perception and associated cognitive processing while engaging with digital displays, the visual content of which can be known and optionally changed in a controlled fashion. For example, this enables reproducible test runs of multiple users through a prepared environment to learn about the cognitive performances and differences in attention and how it is influenced by visual or other signals. This enables data-driven optimizations and enhancements for better user engagement and comprehension as well as real-time feedback and adaptive features based on the user's cognitive states and attentional distribution.

These examples emphasize the applicability of the invention within specific contexts and use cases, highlighting its potential value for developers and end-users alike. By incorporating the novel methodologies described above into a mobile headset designed for computer display interaction, this application provides an innovative solution to address real-world challenges related to user engagement, cognitive processing, and visual perception.

Referring now to the drawings, wherein the figures are for purposes of illustrating the present invention and not for purposes of limiting the same,

Fig. 1 schematically shows an exemplary processing scheme implementing the present method and system in an VR/MR setup; and
Fig. 2 schematically shows a second embodiment using AR glasses.

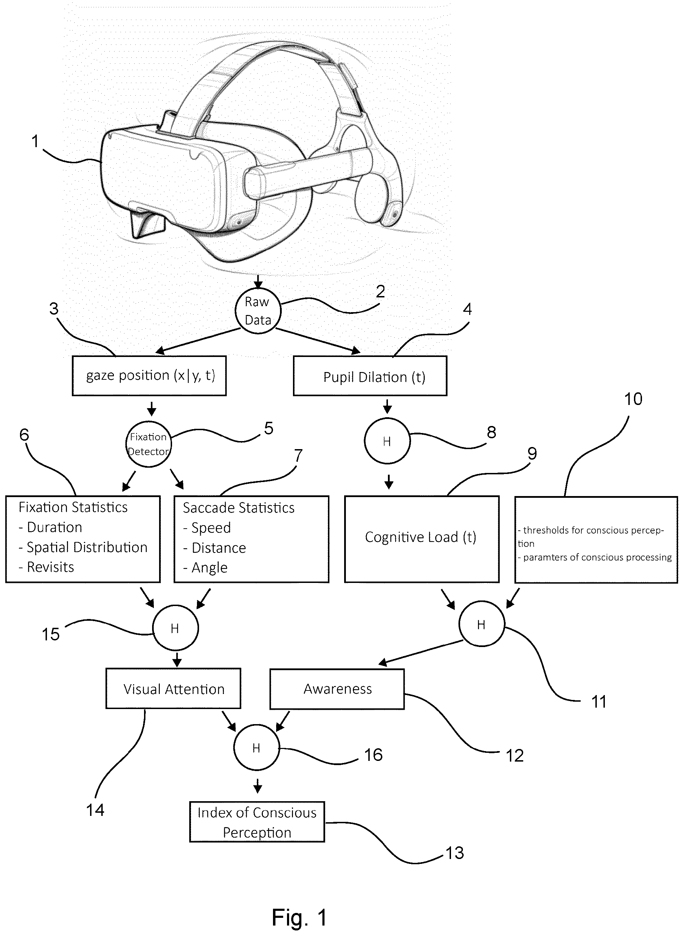

The processing scheme shown in Fig. 1 combines multiple independently determined parameters into a measure of targeted visual attention, more specifically a joint description of visual attention and awareness. The multiple parameters are features describing gaze behavior via fixations and saccades as well as cognitive load (as defined in the introduction together with citations). The resulting measure can be used as a score of conscious perception which can be interpreted for individual areas of interest or objects.

For this purpose, the exemplary method realizes a multi-modal integration of features for modelling conscious perception via combination of metrics of visual attention (location and duration of eye gaze), cognitive load (pupillometric analytics), and features from cognitive psychology (thresholds and requirements for consciousness of visual perception).

The method disclosed in Fig. 1 uses established eye tracking information of the gaze position and derived interpretations of gaze behavior statistics (fixations and saccades) to describe the location of visual attention in a spatial environment (VR, MR). This aspect of WHERE human attention is located, is combined with additional inferences on HOW (quality) human attention is being directed, via analysis of cognitive load from pupil dilation and insights into human perception and awareness as derived from psychological studies.

The measure of targeted attention corresponds to a biomarker. For its determination, eye tracker hardware is used, which provides gaze information (gaze position in (X|Y) or in angular coordinates) and pupil dilation (in mm or in pixel units, corresponding to the size of the pupil), in the present example. In this embodiment, an XR (Extended Reality) headset 1 is used. The XR headset 1 provides integrated eye tracking. Generally, any eye tracking device may be used for the same purpose. The integrated eye tracking system of the XR headset 1 captures series of images of each eye of the wearer of the XR headset 1. These captured images are raw data 2. From the raw data 2, the eye tracking system of the XR headset 1 performs gaze detection 3, which determines for each eye the current gaze position, or - generally - the gaze position x|y as a function of time t, generally referred to as gaze information. In parallel, the eye tracking system of the XR headset 1 performs pupil detection 4 based on the raw data 2 and determines a pupil dilation for each eye - again, as a function of time t. The actual computation of the measure combines analytics of two inputs: gaze behavior analytics, and mental effort / cognitive load. Gaze behavior analytics comprises fixation detection, fixation statistics, and saccade statistics.

Based on the gaze information, a fixation detector 5 determines a gaze metric to distinguish between fixations and saccades and trigger the adjacent computation of the associated statistical features. This detection is built upon the comparison of consecutive frames, monitoring the delta between gaze points to observe whether consecutive gaze points meet the conditions of a fixation which are: angular distance of gaze points in relation to the geometric center of the collected gaze points does not exceed the threshold $\delta$ ($\delta$ is set to 1.5° as default according to literature, but may be adapted according to the sensitivity of the respective eye tracking device); duration of the accumulated time period of consecutive gaze points which meet the distance condition exceeds the time threshold of 100 ms, in this example.

If both conditions are met, the fixation detector 5 passes the gaze information to a fixation module 6. The fixation module 6 computes the following statistics (which represent further gaze metrics) for fixation $\Phi_i$ starting from time $t_0$ of the first frame in the analyzed window of fixation frames.

Fixation duration $t_i$: time window from the beginning of the fixation $t_o$ to the current timeframe $t_j$

$$t_i = t_j - t_0$$

Fixation location: Geometric centroid of all analyzed fixation gaze points

$$(\overline{x_i}, \overline{y_i}) = \left( \frac{1}{n} \sum_{i=0}^{n} x_j , \frac{1}{n} \sum_{i=0}^{n} y_j \right)$$

Revisits: an integer counter $\phi$ which is increased if the geometric centroid (i.e. the fixation location defined above) of a subsequent fixation falls in the close perimeter of the fixation location of the present fixation

$$\phi_i += 1$$

Spatial Distribution: The nearest neighbor index (NNI) is an established measure of spatial clustering. It compares the observed mean neighbor distances of fixations with a hypothetical maximum of dispersed observations given the area of the zone. This allows an interpretation of how clustered gaze behavior is (interpretation towards focused behavior) or how dispersed (interpretation towards search behavior).

$$NNI_i = \frac{N\overline{N}D_i}{\left( 0.5 * \sqrt{\frac{A_i}{n_i}} \right)}$$

These features are combined into the fixation descriptor $\Phi_i(t_i, \overline{x_i}, \overline{y_i}, \phi_i, NNI_i)$ which is stored in memory in a list for later analysis and interpretation.

If no fixation is detected by the fixation detector 5, the gaze points are interpreted as part of a saccade and the fixation detector 5 passes the gaze information to a saccade module 7. For a saccade $\Pi_k$, the saccade module 7 computes the following features (yet further gaze metrics):

Saccade duration: time window from the beginning of the saccade $t_0$ to the current timeframe $t_l$

$$t_k = t_l - t_0$$

Saccade length: The sum of the traveled saccade path

$$l_k = \sum_{l=1}^{n} \sqrt{(x_l - x_{l-1})^2 + (y_l - y_{l-1})^2}$$

Saccade distance: distance of the direct line between onset and offset of the saccade, representing the actual effective saccade.

$$d_k = \sqrt{(x_l - x_0)^2 + (y_l - y_0)^2}$$

Saccade efficiency: Saccade efficiency is computed as the ratio between saccade length and distance

$$e = \frac{1}{N} \sum_{k=1}^{N} \frac{d_k}{l_k}$$

Regarding modeling mental effort and cognitive load, the measure of targeted attention builds upon previously developed cognitive load algorithms as published and described in earlier academic publications (see publications cited in the introduction, specifically Gollan et al in COGNITIVE, 2016).

In short, a compensation module 8 analyzes pupil dilation an received from the eye tracking system by exploiting a first person field of view camera as brightness sensor and feed this brightness data into an empiric model of pupillary light reflex to compute how the human pupil behaves with respect to the environmental brightness. This allows the separation of brightness and cognitive effects.

Based on this compensated pupil dilation data, a cognitive load module 9 applies a mathematical model of the Task-Evoked Pupil Response (TEPR; see Hoeks, B., & Levelt, W. J. (1993). Pupillary dilation as a measure of attention: A quantitative system analysis. Behavior Research methods, instruments, & computers, 25(1), 16-26.) via a multi-parameter curve matching optimization process to obtain a real-time estimation of cognitive load A(t), normalized to a score between o to 1.

A threshold configuration unit 10 provides filter configuration for a subsequent cognitive load filter 11. The cognitive load filter 11 applies a rule-based model to the estimated cognitive load, with configuration received from the threshold configuration unit 10. Said model is based on insights from cognitive science related to the possibility of consciousness of perception. According to this model, conscious perception of content requires a minimum fixation duration $t_i$ of - for example -

$$t_i > \tau,$$

where $\tau = 200ms$
resulting in a binary function of

$$\varphi(t_i) = \begin{cases} 1 \ if \ t_i > \tau \\ 0 \ if \ t_i \le \tau \end{cases}$$

The combination of both scores by the cognitive load filter 11 results in the modeled level of Awareness 12, abbreviated as $\Upsilon(t, i)$ of a person at time t:

$$\Upsilon(t, i) = \Lambda(t) * \varphi(t_i)$$

The Conscious Perception Index (CoPI) 13, abbreviated as $\Omega_i$ - which provides a targeted measure of visual attention in this example - consists fundamentally of two parameters: the location of the associated fixation i (x|y); and the score of conscious perception probability $\Omega_i \in [0; 1]$. Apart from the level of awareness 12 discussed above, it depends on the level of visual attention 14, which is determined by the attention filter 15 based on weighted, normalized parameters (weights: $\alpha, \beta, \gamma, \delta$, see below) associated with the saccade efficiency, the fixation duration, the geometric distribution of fixations and the revisits of locations.

The scale of the individual weights has been determined qualitatively in the scope of current R&D projects in the field of pilot training and training of medical professionals, to produce the best coherence between modeling results, task performance and self-reports of study subjects. The consciousness of perception of the study participants was evaluated based on the established NASA Task Load Index for load analytics, a questionnaire on their immersiveness of the training experience (in VR), and a questionnaire for self-evaluation of their performance to extract reference qualitative consciousness scores. These scores were used to fine-tune the parameter weights to optimize the fit between CoPI and reference scores. Based on these inputs, the parameters were defined as:

$$\alpha = 0.15$$

$$\beta = 0.4$$

$$\gamma = 0.2$$

$$\delta = 0.25$$

In different contexts, these parameters may need to be adapted to provide realistic representation of observed consciousness patterns. This is due to the fact that different application scenarios (e.g. interaction with an aviation flightdeck, performance of surgeries or high-speed sport applications) show different characteristic requirements towards gaze behavior - specifically on the parameters of fixation duration and geometric distribution. Translating these scores to completely different use-cases may require a re-evaluation of the weights, according to the previously described process.

Applying these weights to the overall calculation results in, with the first factor corresponding to the visual attention 14 and the second factor representing the level of awareness 12:

$$\Omega_i = \frac{(0.15 * e^{\phantom{-}} + 0.4 * (1 - 3^{-3t_i}) + 0.2 * \left(\frac{2}{1 + e^{-NNI_i}} - 1\right) + 0.25 * \left(\sum_{l=1}^{\phi_i} \frac{1}{l}\right)}{(0.15 + 0.4 + 0.2 + 0.25)} * \Upsilon(t, i)$$

$$\text{at location } (\bar{x}_i | \bar{y}_i)$$

The integration module 16 receives the visual attention 14 and the level of awareness 12 and combines them to determine the CoPI 13 for a given location.

To summarize, the CoPI 13, i.e. which is the targeted attention as discussed above, provides insights into:

- whether conscious acquisition and processing of information has been possible if conscious perception was possible,
- an estimation of the probability of conscious perception
- a quantitative measure of the intensity of engagement and perception
- the spatial distribution of the conscious perception (conscious perception heatmap)
- the mapping of evaluation of conscious perception onto objects/areas of interest.the mapping of evaluation of conscious perception onto objects/areas of interest.

For each fixation i, the CoPI value is recorded and collected in a memory and continuously updated with new fixations as they are detected. The results may be output for example in one or more of the following ways:

- For each frame (of captured images), an associated CoPI value of an active fixation is stored in an output file, e.g. a text file (as comma-separated values).
- A list of all fixations with the associated CoPI value is stored in an output file (as comma-separated values).
- The fixations and associated CoPI values may be rendered in a heatmap and displayed on a graphic display, e.g. as a live overlay of a screen.

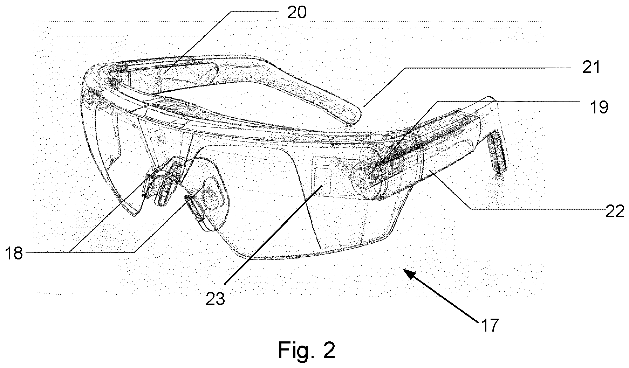

Fig. 2 shows a second embodiment with a different type of eye tracking device, namely a pair of AR glasses 17. The AR glasses 17 provide an eye tracking system and a CoPI evaluation system according to the present disclosure. The eye tracking system comprises eye tracking sensors in the form of two eye cameras 18. Each eye camera 18, left and right, is configured to capture images of the respective eye of the user. A world camera 19 is configured to capture the field of view of the user, and suitable to provide baseline information regarding the current brightness. A computing unit 20 comprises a processor (CPU) and memory (RAM) and is connected to the eye cameras 18, the world camera 19, as well as a wired USB interface 21 and a wireless communication module 22. The computing unit 20 stores a computer program comprising instructions which, when executed, implement the tasks of the modules described in connection with Fig. 1, i.e. the fixation detector 5, the fixation module 6, the saccade module 7, the compensation module 8, the cognitive load module 9, the threshold configuration unit 10, the cognitive load filter 11, the attention filter 15 and the integration module 16. The AR glasses 17 are equipped with a USB interface for charging an integrated rechargeable battery as well as to provide access to the memory and storage of the AR glasses 17. The determined CoPI results may be communicated to an external service via the wireless communication module 22, for example, and/or an updated CoPI heatmap may be displayed to the user via an integrated see-through display 23.

**Claims**

1. A computer-implemented method for monitoring visual perception and associated cognitive processing of a user, the method comprising:

   capturing a series of images of an eye;
   selecting a first subset of the captured images;
   detecting the position of the pupil in each image in the first subset;
   selecting a second subset of the captured images;
   detecting the size of the pupil in each image in the second subset;
   determining a cognitive load metric based on the detected sizes;
   determining at least two gaze metrics of an eye gaze behavior based on the detected positions, wherein one of the at least two gaze metrics is a direction of gaze; and
   determining a measure of targeted attention based on the cognitive load metric and the at least two gaze metrics.

2. The method according to claim 1, **characterized in that** the measure of targeted attention is proportional to the cognitive load metric.

3. The method according to claim 1 or 2, **characterized in that** one of the at least two gaze metrics is a fixation duration and the measure of targeted attention is proportional to a step function of the fixation duration with a threshold higher than 50 milliseconds, preferably higher than 200 milliseconds.

4. The method according to any one of claims 1 to 3, **characterized in that** one of the at least two gaze metrics is a fixation duration and the measure of targeted attention is proportional to a sigmoid function of the fixation duration.

5. The method according to any one of claims 1 to 4, **characterized in that** one of the at least two gaze metrics is a count of revisits, wherein a revisit is a fixation within a threshold distance of a previous fixation.

6. The method according to claim 5, **characterized in that** the measure of targeted attention is proportional to a sigmoid function of the count of revisits.

7. The method according to any one of claims 1 to 6, **characterized in that** one of the at least two gaze metrics is a measure of clustering of fixations, in particular a nearest neighbor index of fixations.

8. The method according to claim 7, **characterized in that** the measure of targeted attention is proportional to a sigmoid function of the measure of clustering of fixations.

9. The method according to any one of claims 1 to 8, **characterized in that** one of the at least two gaze metrics is a saccade efficiency.

10. The method according to claim 9, **characterized in that** the measure of targeted attention is proportional to the saccade efficiency.

11. The method according to any one of claims 1 to 10, **characterized by** aggregating the measure of targeted attention over an integration time window to generate a heatmap of targeted attention.

12. The method according to claim 11, **characterized by** determining a global maximum of the heatmap and comparing the global maximum to a predefined attention threshold and associating a current attention target corresponding to the location of the global maximum to the integration time window responsive to detecting that the global maximum is greater than the predefined attention threshold.

13. A computer program product comprising instructions which, when the program is executed by a computer, cause the computer to carry out the method of any one of claims 1 to 12.

14. A mobile headset (1; 17) comprising an eye tracking sensor and a data processing device comprising means for carrying out the method of any one of claims 1 to 12.

15. Use of a method according to any one of claims 1 to 12, in particular within a mobile headset according to claim 14, while the user is interacting with visual content presented on a computer display.

**Amended claims in accordance with Rule 137(2) EPC.**

1. A computer-implemented method for monitoring visual perception and associated cognitive processing of a user, the method comprising:

capturing a series of images of an eye;
selecting a first subset of the captured images;
detecting the position of the pupil in each image in the first subset;
selecting a second subset of the captured images;
detecting the size of the pupil in each image in the second subset;
determining a cognitive load metric based on the detected sizes;
determining at least two gaze metrics of an eye gaze behavior based on the detected positions, wherein one of the at least two gaze metrics is a direction of gaze and another one of the at least two gaze metrics is a saccade efficiency; and
determining a measure of targeted attention based on the cognitive load metric and the at least two gaze metrics.

2. The method according to claim 1, **characterized in that** the measure of targeted attention is proportional to the cognitive load metric.

3. The method according to claim 1 or 2, **characterized in that** one of the at least two gaze metrics is a fixation duration and the measure of targeted attention is proportional to a step function of the fixation duration with a threshold higher than 50 milliseconds, preferably higher than 200 milliseconds.

4. The method according to any one of claims 1 to 3, **characterized in that** one of the at least two gaze metrics is a fixation duration and the measure of targeted attention is proportional to a sigmoid function of the fixation duration.

5. The method according to any one of claims 1 to 4, **characterized in that** one of the at least two gaze metrics is a count of revisits, wherein a revisit is a fixation within a threshold distance of a previous fixation.

6. The method according to claim 5, **characterized in that** the measure of targeted attention is proportional to a sigmoid function of the count of revisits.

7. The method according to any one of claims 1 to 6, **characterized in that** one of the at least two gaze metrics is a measure of clustering of fixations, in particular a nearest neighbor index of fixations.

8. The method according to claim 7, **characterized in that** the measure of targeted attention is proportional to a sigmoid function of the measure of clustering of fixations.

9. The method according to any one of claims t to 8, **characterized in that** the measure of targeted attention is proportional to the saccade efficiency.

10. The method according to any one of claims 1 to 9, **characterized by** aggregating the measure of targeted attention over an integration time window to generate a heatmap of targeted attention.

11. The method according to claim 10, **characterized by** determining a global maximum of the heatmap and comparing the global maximum to a predefined attention threshold and associating a current attention target corresponding to the location of the global maximum to the integration time window responsive to detecting that the global maximum is greater than the predefined attention threshold.

12. A computer program product comprising instructions which, when the program is executed by a computer, cause the computer to carry out the method of any one of claims 1 to 11.

13. A mobile headset (1; 17) comprising an eye tracking sensor and a data processing device comprising means for carrying out the method of any one of claims 1 to 11.

14. Use of a method according to any one of claims 1 to 11, in particular within a mobile headset according to claim 13, while the user is interacting with visual content presented on a computer display.

1

Raw Data 2

3 gaze position (x|y, t)     Pupil Dilation (t) 4

10

6 Fixation Detector 5

8 H

7

9

Fixation Statistics
- Duration
- Spatial Distribution
- Revisits

Saccade Statistics
- Speed
- Distance
- Angle

Cognitive Load (t)

- thresholds for conscious perception
- paramters of conscious processing

15 H

H

Visual Attention     Awareness

11

14

H 16

12

Index of Conscious Perception

13

Fig. 1

EP 4 613 176 A1

Fig. 2

13

Europäisches
Patentamt

European
Patent Office

Office européen
des brevets

# EUROPEAN SEARCH REPORT

**Application Number**

EP 24 16 1204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Jeff Klingner: "MEASURING COGNITIVE LOAD DURING VISUAL TASKS BY COMBINING PUPILLOMETRY AND EYE TRACKING", , 1 May 2010 (2010-05-01), XP055256289, Retrieved from the Internet: URL:http://graphics.stanford.edu/papers/klingner-dissertation/klingner-dissertation.pdf [retrieved on 2016-03-08] | 1-3,5,7, 9,10, 13-15 | INV. A61B3/11 A61B3/113 |
| Y | * pages 7-88 * | 4 | |
| X | SKARAMAGKAS VASILEIOS ET AL: "Review of Eye Tracking Metrics Involved in Emotional and Cognitive Processes", IEEE REVIEWS IN BIOMEDICAL ENGINEERING, IEEE, USA, vol. 16, 16 March 2021 (2021-03-16), pages 260-277, XP011931940, ISSN: 1937-3333, DOI: 10.1109/RBME.2021.3066072 [retrieved on 2021-03-17] * page 261, column 2, paragraph II. - page 272, column 2 * | 1,2,9, 10,13-15 | |
| X | KR 2022 0157643 A (UNIV EWHA IND COLLABORATION [KR]) 29 November 2022 (2022-11-29) | 1,2,5,7, 11,13,15 | |
| Y | * paragraphs [0012] - [0059]; figures 1-3b * | 6,8,12 | |
| X | US 2020/155053 A1 (BERNSTEIN AMIT [IL]) 21 May 2020 (2020-05-21) * paragraphs [0063], [0095] - [0097] * | 1-3,13, 15 | |

**TECHNICAL FIELDS SEARCHED (IPC)**

A61B

-/--

The present search report has been drawn up for all claims

| Place of search | Date of completion of the search | Examiner |
|---|---|---|
| Munich | 24 July 2024 | Mäki-Mantila, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

**EUROPEAN SEARCH REPORT**

Application Number

EP 24 16 1204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/195222 A1 (KHADERI SYED KHIZER RAHIM [US] ET AL) 22 June 2023 (2023-06-22) | 1-3, 13-15 | |
| Y | * paragraphs [0216], [0230] - [0251], [0431]; figures 1-3 * | 4,6,8 | |
| | ----- | | |
| Y | CN 109 799 908 A (UNIV SOUTHEAST; BEIJING REMOTE SENSING INF INST) 24 May 2019 (2019-05-24) * paragraph [0043] * | 12 | |
| | ----- | | |

TECHNICAL FIELDS
SEARCHED     (IPC)

The present search report has been drawn up for all claims

| Place of search | Date of completion of the search | Examiner |
|---|---|---|
| Munich | 24 July 2024 | Mäki-Mantila, M |

EPO FORM 1503 03.82 (P04C01)

## ANNEX TO THE EUROPEAN SEARCH REPORT
## ON EUROPEAN PATENT APPLICATION NO.

EP 24 16 1204

This annex lists the patent family members relating to the patent documents cited in the above-mentioned European search report.
The members are as contained in the European Patent Office EDP file on
The European Patent Office is in no way liable for these particulars which are merely given for the purpose of information.

24-07-2024

| Patent document cited in search report | Publication date | Patent family member(s) | | Publication date |
|---|---|---|---|---|
| KR 20220157643 A | 29-11-2022 | NONE | | |
| US 2020155053 A1 | 21-05-2020 | NONE | | |
| US 2023195222 A1 | 22-06-2023 | AU | 2017248362 A1 | 22-11-2018 |
| | | AU | 2017248363 A1 | 22-11-2018 |
| | | CA | 3020390 A1 | 12-10-2017 |
| | | CA | 3020394 A1 | 12-10-2017 |
| | | CN | 109640785 A | 16-04-2019 |
| | | CN | 109690384 A | 26-04-2019 |
| | | EP | 3439533 A1 | 13-02-2019 |
| | | EP | 3440494 A1 | 13-02-2019 |
| | | JP | 2019513516 A | 30-05-2019 |
| | | JP | 2019519053 A | 04-07-2019 |
| | | KR | 20190026651 A | 13-03-2019 |
| | | KR | 20190027354 A | 14-03-2019 |
| | | US | 2017290504 A1 | 12-10-2017 |
| | | US | 2017293356 A1 | 12-10-2017 |
| | | US | 2019391638 A1 | 26-12-2019 |
| | | US | 2023195222 A1 | 22-06-2023 |
| | | WO | 2017177187 A1 | 12-10-2017 |
| | | WO | 2017177188 A1 | 12-10-2017 |
| CN 109799908 A | 24-05-2019 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7438418 B

**Non-patent literature cited in the description**

- Tracking visual search demands and memory load through pupil dilation. *Journal of Vision*, 2020, vol. 20 (6), 1-19
- **GOLLAN et al.** Demonstrator for Extracting Cognitive Load from Pupil Dilation for Attention Management Services. *UBICOMP/ISWC '16 ADJUNCT*, 12 September 2016
- **GOLLAN** ; **FERSCHA**. Modeling Pupil Dilation as Online Input for Estimation of Cognitive Load in non-laboratory Attention-Aware Systems. *COGNITIVE*, 2016
- **GOLLAN et al.** *COGNITIVE*, 2016
- **HOEKS, B.** ; **LEVELT, W. J.** Pupillary dilation as a measure of attention: A quantitative system analysis. *Behavior Research methods, instruments, & computers*, 1993, vol. 25 (1), 16-26